# EUROPÄISCHE PATENTSCHRIFT

(11) **EP 1 092 769 B1**
(45) Veröffentlichungstag und Bekanntmachung des Hinweises auf die Patenterteilung: **29.10.2008**
(21) Anmeldenummer: 00117845.8
(22) Anmeldetag: 18.08.2000
(51) Int. Cl.: C12N 15/11, C07K 7/06, C12N 15/62, C07K 16/44

(54) **Polypeptid-Tag's für den Nachweis und die Aufreinigung von Polypeptiden**
Polypeptid-tag's for the detection and purification of polypeptides
Des "tags" polypeptidiques pour la détection et la purification de polypeptides

(30) Priorität: 15.10.1999 DE 19949855; 19.04.2000 DE 10019473
(43) Veröffentlichungstag der Anmeldung: 18.04.2001
(73) Patentinhaber: Fraunhofer-Gesellschaft zur Förderung der angewandten Forschung e.V., 80686 München (DE)
(72) Erfinder: Fischer, Rainer, Dr., 52074 Aachen (DE); Helgers, Yvonne, 52062 Aachen (DE); Hoffmann, Kurt, Dr., 4770 Amel (BE); Holzem, Achim, 52072 Aachen (DE); Monecke, Michael, 52066 Aachen (DE); Nähring, Jörg, 52074 Aachen (DE); Schillerberg, Stefan, Dr., 52072 Aachen (DE)
(74) Vertreter: Becker Kurig Straus

(56) Entgegenhaltungen:
- WO-A-00/23593

## Beschreibung

Die vorliegende Erfindung betrifft Polypeptid-Tag's, die zur Isolierung, zur Aufreinigung sowie zum Nachweis von Polypeptiden verwendet werden können. Die vorliegende Erfindung betrifft zudem Expressionsvektoren, die ein Fusionspolypeptid kodieren, das ein tag54-Epitop und ein gewünschtes Polypeptid umfaßt, sowie die Verwendung derartiger Expressionsvektoren zur Expression der Fusionspolypeptide.

Die Expression von Polypeptiden stellt nach wie vor eine Herausforderung dar. Dies gilt insbesondere dann, wenn das exprimierte Polypeptid ungeachtet des verwendeten Expressionssystems nachgewiesen und aufgereinigt werden soll.

Zur Analyse bzw. Aufreinigung von Polypeptiden wird gegenwärtig die Technik des sogenannten "Epitop-Taggings" eingesetzt, bei dem mittels molekularbiologischer Techniken ein bestimmtes Epitop an ein Polypeptid von Interesse angehängt und das bei der Expression entstehende Fusionspeptid dann mittels gegen das "Tag" gerichteter Antikörper isoliert wird. Dabei wird die Primärsequenz eines beliebigen Polypeptids mit Hilfe rekombinanter Techniken um einige wenige Aminosäuren erweitert, die von dem Antikörper spezifisch erkannt werden. Ausschlaggebend für den Erfolg ist daher das Vorhandensein eines spezifischen und hochaffinen Antikörpers mit bekannter Erkennungssequenz, der die Sequenz ohne Kreuzreaktivität mit anderen Proteinsequenzen spezifisch erkennt.

Als ein derartiges "Tag" wurde bis dato ein Rest aus sechs Histidinresten (His₆) eingesetzt. Dieses "Tag" weist jedoch den Nachteil auf, daß gegen ihn keine hochaffinen Antikörper herstellbar sind und daß die vorhandenen Antikörper mit verschiedenem Proteinmaterial kreuzreaktiv sind.

Eine Aufgabe der vorliegenden Erfindung besteht daher darin, ein Mittel bereitzustellen, das die Nachteile der bekannten "Tags" vermeidet und mit dem Polypeptide exprimiert und spezifisch nachgewiesen werden können.

Diese Aufgabe wird gelöst durch ein Polypeptid-Tag, das die folgende Aminosäuresequenz aufweist:
-Lys-Asp-Trp-Glu-His-Leu- (Seq ID No. 2)
bzw. eine von der vorstehend angegebenen Sequenz abgeleitete Aminosäuresequenz, bei der die dreidimensionale bzw. Tertiärstruktur im wesentlichen erhalten bleibt. Die von der angegebenen Aminosäuresequenz abgeleitete Sequenz kann durch Substitution und/oder Inversionen einer Aminosäure erhalten werden. Gemäß einer bevorzugten Ausführungsform weist das Polypeptid-Tag die folgende Aminosäuresequenz auf: Lys-His-Ile-Lys-Asp-Trp-Glu-His-Leu-Glu-Glu-Phe (Seq ID No. 3) bzw. eine von der vorstehend angegebenen Sequenz abgeleitete Aminosäuresequenz, bei der die dreidimensionale bzw. Tertiärstruktur im wesentlichen erhalten bleibt.

In den Figuren,
Figur 1 zeigt schematisch einen Vektor (pSSH1) für die Expression von Fusionspeptiden in Pflanzen; und
Fig. 2 zeigt schematisch einen Expressionsvektor (pET22b) für Fusionspeptide in Bakterien.

Die hier genannten Erfinder haben nun gefunden, daß sich ein bestimmter Bereich des TMV 54K Proteins (vgl. Padgett et al. "Analysis of a Tobacco Mosaic Virus Strain capable of overcoming N gene-mediated resistance"; Plant Cell 5 (1993), 577-586), insbesondere die Aminosäuresequenz zwischen den Aminosäuren 405 und 410 des TMV 54K Protein hervorragend als "Tag" eignet, da gegen diesen Bereich hochaffine Antikörper hergestellt werden können und eine Kreuzreaktivität mit zellulären Epitopen bakteriellen und eukaryotischen Ursprungs im wesentlichen nicht vorhanden ist.

Um die vorstehend aufgeführten Aminosäuresequenzen als "Tag" für ein Polypeptid von Interesse zu verwenden wird eine das "Tag" kodierende Nukleotidsequenz in einen Expressionsvektor inseriert, die vorzugsweise die folgende Basenfolge aufweist:
AAG GAC TGG GAG CAC (SEQ ID No. 4)
bzw.
AAG CAC ATC AAG GAC TGG GAG CAC CTC GAA GAG TTC (SEQ ID No.5)
bzw. eine von der angegebenen Sequenz abgeleitete Sequenz, die ein erfindungsgemäßes "Tag" kodiert.

In den Expressionsvektor wird zudem eine ein Polypeptid von Interesse kodierende DNA-Sequenz eingefügt, derart, daß beide DNA-Sequenzen ein einheitliches Leseraster ausbilden und ein Fusionspolypeptid hergestellt wird, das das Polypeptid von Interesse und das "Tag" enthält. Dabei kann das "Tag" am N- oder C-terminalen Ende, bzw. auch innerhalb des Fusionspolypeptids vorliegen und ggf. von dem Polypeptid durch einen aus einer oder mehreren Aminosäuren bestehenden Spacer beabstandet sein.

Die Expressionsvektoren enthalten gewöhnlich die für sie üblichen Elemente, wie Promotor, Polylinker zur Erleichterung der Klonierung von Fremdsequenzen, Leadersequenzen zur Steuerung des Fusionspeptids aus der Zelle heraus usw.. Die Vektoren werden dabei unter Berücksichtigung der Anforderungen des gewünschten Expressionssystems ausgewählt. Für eine Expression in E. coli können beispielsweise die Vektoren pGEMEX, pUC-Derivate, pGEX-2T, pET-Derivate, pQE8 verwendet werden, die bereits alle weit verbreitet als Expressionsvektoren zum Einsatz kommen.

Für Hefen sind beispielsweise die Vektoren pY100, Ycpad1, pPIC9K und pPICz einsetzbar, während für eine Expression in tierischen Zellen die Vektoren pKCR, pEFBOS, cDM8 und pCEV4 verwendet werden können. Für eine Expression in Pflanzen kann der Vektor pAL76 sowie pSS-Derivate (siehe beispielsweise Kay R. et al, Science 236 (1987), 1299-1302) eingesetzt werden, während eine Expression in Insektenzellen mittels beispielsweise des Baculovirus-Expressionsvektor pAcSGNT-A erzielt werden kann.

Der Fachmann kann auf Grundlage seines allgemeinen Fachwissens die jeweiligen DNA-Sequenzen für das Polypeptid-Tag bzw. das Polypeptid von Interesse in den Expressionsvektor inserieren, so daß eine Expression des Fusionspeptids erreicht bzw. optimiert werden kann. Dabei kann es bevorzugt sein, dass die das Polypeptid-Tag kodierende DNA-Sequenz vor dem Einbringen der DNA für das Polypeptid in den Expressionsvektor inseriert wird. Ferner ist dem Fachmann klar, daß aufgrund der Degeneration des genetischen Codes die DNA in der Weise verändert werden kann, um beispielsweise eine Kodon-optimierte DNA-Sequenz für das jeweilige Expressionssystem bereitzustellen.

Dem Fachmann sind weiter Verfahren und Zellen bekannt, um Fusionspolypeptide zu exprimieren. Beispiele derartiger Zellen umfassen die E. coli Stämme HB101, DH1, JM101, JM109 und BL21, die Hefen Saccharomyces cerevisae und Pichia pastoris, die tierischen Zelllinien L 3T3, FM3A, CHO, COS, Vero und HeLa, sowie die Insektenzelllinie Sf9. Ferner können Monocotyledoneae und Dicotyledoneae, sowohl als Pflanze als auch in Zell-Suspensionskultur verwendet werden.

Weiterhin liefert die vorliegende Erfindung einen gegen das erfindungsgemäße Polypeptid gerichteten Antikörper.

Mit Hilfe von Antikörpern, die spezifisch gegen den erweiterten Proteinanteil gerichtet sind, kann beispielsweise das Molekulargewicht eines Proteins bestimmt werden, seine zelluläre Lokalisation, posttranslationale Modifizierungen oder Wechselwirkungen mit anderen Faktoren, ohne dass proteinspezifische Antikörper vorhanden sein müssen, sowie eine Reinigung von Proteinen einfach durchgeführt werden (Affinitätschromatographie).

Der Antikörper kann ein polyklonaler oder monoklonaler Antikörper oder ein das "Tag" bindendes Fragment davon sein, wobei ein monoklonaler Antikörper bevorzugt wird. Dieser kann nach üblichen, dem Fachmann bekannten Verfahren hergestellt werden. Sollen polyklonale bzw. monoklonale Antikörper hergestellt werden, dann ist es vorteilhaft, Tiere, insbesondere Kaninchen zum Erhalt polyklonaler Antikörper, sowie Mäuse zum Erhalt monoklonale Antikörper mit dem entsprechendem Fusionspolypeptid oder einem Fragment davon, das das entsprechende Epitop enthält, bzw. mit dem gereinigten Epitop, zu immunisieren. Ein weiteres Boosten der Tiere kann mit den gleichen Antigenen erfolgen. Zudem können andere, das erfindungsgemäße Polypeptid-Tag enthaltende Fusionspeptide oder ein Gemisch aus dem zuerst eingesetzten Fusionspolypeptid und einem oder mehreren neuen Fusionspolypeptiden zum Boosten verwendet werden. Die so erhaltenen polyklonalen Antikörper können dann aus dem Serum der Tiere gewonnen werden.

Zum Erhalt monoklonaler Antikörper werden Milzzellen der immunisierten Tiere gemäß bekannter Methoden mit Myelomzellen fusioniert.

Darüber hinaus kann ein erfindungsgemäßer Antikörper synthetisch sein, wobei ggf. Teile, die für die Erkennung des erfindungsgemäßen Polypeptid-Tags nicht notwendig sind, ganz oder teilweise fehlen bzw. diese Teile durch andere ersetzt sind, welche dem Antikörper weitere günstige Eigenschaften verleihen.

Zur Herstellung synthetischer Antikörper kann beispielsweise von wie vorstehend erhaltenen, monoklonalen Antikörpern ausgegangen werden. Hierzu können die Antigen-Bindungsregionen der monoklonalen Antikörper analysiert und die für Erkennung notwendigen und nicht notwendigen Teile identifiziert werden. Die notwendigen Teile können dann modifiziert und die nicht notwendigen ganz oder teilweise eliminiert bzw. durch Teile ersetzt werden, die den Antikörpern weitere günstige Eigenschaften verleihen. Auch können Teile außerhalb der Bindungsregionen der Antikörper modifiziert, eliminiert oder ersetzt werden.

Die erfindungsgemäßen Antikörper zeichnen sich dadurch aus, dass sie über das erfindungsgemäße Polypeptid-Tag beliebige Fusionspolypeptide erkennen und binden können. Die Antikörper eignen sich daher zum schnellen Nachweis der Expression derartiger Fusionspolypeptide, welcher in beliebigen Verfahren durchgeführt werden kann, insbesondere in einem Western Blot, einem ELISA, einer Immunpräzipitation oder einer Immunfluoreszenz. Hierzu können die erfindungsgemäßen Antikörper markiert sein oder in Kombination mit markierten, gegen diese gerichteten Antikörpern eingesetzt werden.

Die erfindungsgemäßen Antikörper können dazu verwendet werden, die entsprechenden Fusionspolypeptide aus beispielsweise Zellextrakten zu reinigen. Eine Bindung der Antikörper an das "Tag" kann dabei in einem weiten pH-Bereich erfolgen, beispielsweise in einem Bereich von etwa pH 3 bis etwa 10.

Weiterhin liefert die vorliegende Erfindung einen Kit, der einen Expressionsvektor enthält, mit dem ein gewünschtes Polypeptid in einem bestimmten System exprimiert werden kann. Der Expressionsvektor enthält dabei vorzugsweise bereits die DNA-Sequenz für das erfindungsgemäße Polypeptid-Tag, so daß der Fachmann nur noch das Polypeptid von Interesse in den Expressionsvektor einbringen muß. Alternativ kann die das Polypeptid-Tag kodierende DNA-Sequenz separat vorgesehen sein, so daß der Anwender entscheiden kann, an welcher Stelle des resultierenden Fusionspolypeptids das "Tag" vorkommen soll. Weiterhin enthält der Kit einen gegen das erfindungsgemäße Tag gerichteten Antikörper sowie übliche Hilfsstoffe, wie Puffer, Träger, Marker-Verbindungen und Kontrollen.

Die Erfindung wird nun unter Bezugnahme auf die Beispiele weiter erläutert. Wo nicht spezifisch erläutert kamen Techniken und Bedingungen zum Einsatz, wie in Sambrook, J., et al. (1989). Molecular cloning: A laboratory manual, 2nd Ed. (Cold Spring Harbor Laboratory, Cold Spring Harbor, N.Y.) angegeben.

### Beispiel 1

### Herstellung rekombinanter Antikörper

Mittels Hybridomatechnologie (Köhler G. and Milstein C., Nature 256 (1975), 52-53 Continuous Cultures Of Fused Cells Secreting Antibody Of Predefined Specificity) wurde gegen das virale TMV 54K Protein ein monoklonaler Antikörper, mAk 54-1, generiert. Zur Immunisierung wurden weibliche Balb/c Mäuse verwendet, wobei als Antigen ein aus transformierten E. coli Zellen gewonnenes Fusionsprotein von TMV 54K mit GST (Glutathion-S-transferase) eingesetzt wurde.

Nach Isolierung der mRNA aus Antikörper sezernierenden Hybridomazellinien gemäß herkömmlicher Techniken wurden die variablen Domänen der schweren Kette (V_{H}) und der leichten Kette (V_{L}) mittels RT-PCR kloniert, wobei die folgenden Oligonukleotide verwendet wurden:

### V_{H} Amplifikation

### 5'- Primer

### 3'-Primer

5'- TGM RGA GAC GGT GAC CAG RGT C - 3' (SEQ ID No. 7)

### V_{L} Amplifikation

### 5'-Primer

### 3'-Primer

wobei nach IUPAC Konvention von 1984 folgendes gilt:
G: Guanin; A: Adenin; T: Thymin; C: Cytosin
R: Adenin oder Guanin
Y: Thymin oder Cytosin
W: Adenin oder Thymin
S: Guanin oder Cytosin
M: Adenin oder Cytosin
K: Guanin oder Thymin
H: Adenin oder Thymin oder Cytosin
B: Guanin oder Cytosin oder Thymin
V: Guanin oder Adenin oder Cytosin
D: Guanin oder Adenin oder Thymin
N: Guanin oder Adenin oder Thymin oder Cytosin

Die so erhaltenen Nukleotidsequenzen wurden in Form von sogenannten "Einzelketten-Antikörpern" (scFv) in E. coli exprimiert (Winter G. and Milstein C., Nature 349 (1991), 293-299).

Anschließend wurde sowohl die Sequenz des monoklonalen Antikörpers als auch des scFv-Antikörpers aufgeklärt (scFv54-1), beziehungsweise überprüft, wobei kein Spezifitätsunterschied gezeigt werden konnte.

### Beispiel 2

### Identifikation des erkannten Epitops

Gemäß dem Verfahren von Cortese et al. ("Identification of biologically active peptides using random libraries displayed on phage", Curr. Opin. Biotechnol. 6 (1995), 73-80) wurde eine Phagenbibliothek angelegt, um mittels des sogenannten Peptid-Displays lineare als auch strukturelle Epitope zu identifizieren. Die angelegten Phagen-Bibliotheken exprimieren ein unbestimmtes Peptid mit 9 Aminosäuren am N-terminus des Gens VIII von Lambda. Eine Bibliothek präsentierte dabei lineare Peptide, die andere ringförmig geschlossene Peptide.

Um Peptide zu identifizieren, die von dem in Beispiel 1 erhaltenen monoklonalen Antikörper mAk 54-1 erkannt werden, wurden die Bibliotheken mit dem affinitätsgereinigten monoklonalen Antikörper (20 µg pro Selektionsrunde) unter Verwendung eines Standardprotokolls hybridisiert (Cortese et al., supra). In der ersten Runde der Selektion wurden 5x10¹² Phagen in 1 ml PBS mit immobilisiertem Antigen (16 Std., 4°C) inkubiert. Nach Waschen (15 x 4 ml PBST und 5 x 4 ml PBS) wurden bindende Phagen mit 1 ml Glycine-HCl pH 2.2 und 0.1% (w/v) BSA (10min, 20°C) eluiert, mit 60 µl 2M Tris neutralisiert und anschließend zur Infektion von E. coli benutzt. Der Phagentiter wurde anschießend durch Ausplattieren von 100 µl infizierter Zellen auf 2x TY-amp Platten bestimmt. Anreicherungsfaktoren wurden anhand einer parallelen BSA-Kontrolle bestimmt. Monoklonale Phagen der dritten Selektionsrunde wurden auf ihre Reaktivität im Phagenelisa hin überprüft.

Die so erhaltenen, positiv selektierten Phagen wurden sequenziert. Die jeweils erhaltenen Sequenzen wurden verglichen und die Konsensus-Sequenz wurde ermittelt.

Die Ergebnisse sind in Tabelle I zusammengefaßt:

**Tabelle 1**

| | 402 | 403 | 404 | 405 | 406 | 407 | 408 | 409 | 410 | 411 | 412 | 413 |
|---|---|---|---|---|---|---|---|---|---|---|---|---|
| 54K-protein: | K | H | I | **K** | **D** | **W** | **E** | **H** | **L** | E | E | F |
| | | | | | | | | | | | | |
| A2/A9/B8/C9: | | K | R | **K** | **D** | G | **E** | **H** | **W** | L | | |
| A6/B5/F8/H1: | R | Q | A | **K** | **S** | **W** | **S** | **S** | **L** | | | |
| G5: | Y | Q | A | **K** | **E** | **W** | **S** | **N** | **L** | | | |
| H10: | | | | **K** | **D** | **W** | **E** | **H** | **R** | V | P | S |
| | | | | | | | | | | | | |
| Konsensus-sequenz : | | | | **K** | **D** | **W** | **E/S** | **H** | **L** | | | |

Bei einem Vergleich der Konsensussequenz gemäß Tabelle I mit der Sequenz des TMV-Polypeptids 54K konnte die entsprechende, vom mAK 54-1 erkannte Stelle im TMV 54K Polypeptid abgeleitet werden.

### Beispiel 3

### Inkorporation des Epitops in rekombinante Proteine und Nachweis

Nach der in Beispiel 2 beschriebenen Bestimmung des von mAk 54-1 in TMV 54K erkannten Epitops wurden die DNA-Sequenzen gemäß dem genetischen Code abgeleitet und entsprechende Oligonukleotide in einer klassischen Polymerase-Kettenreaktion zur Amplifikation des tag54-Epitops verwendet.

Diese Epitope wurden zur Verwendung als Detektionsmittel für von Pflanzen und Bakterien abgeleitete rekombinante Proteine in handelsübliche Vektoren eingebaut.

### a. Expression in Pflanzen

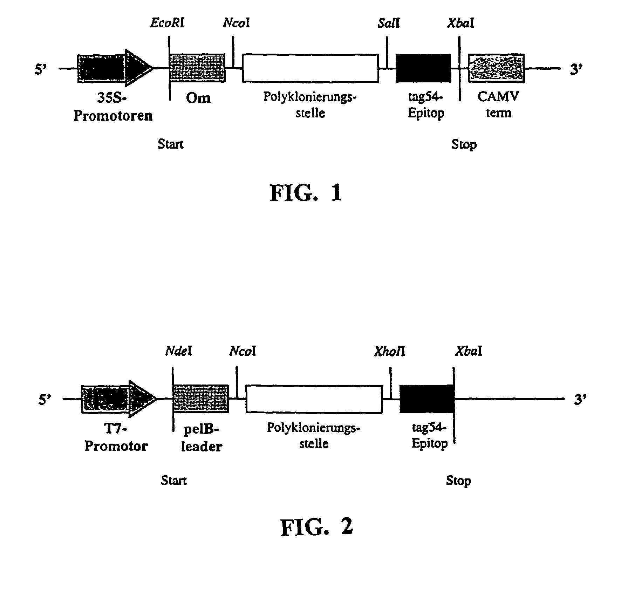

Der Epitop tag-54 wurde "in frame" an den C-Terminus des Antikörperfragmentes scFv24 fusioniert. Dazu wurde das C-terminale c-myc und His6-Fragment aus dem Vektor pscFv24CM (Zimmermann et. al, Molecular Breeding 4 (1998), 369-379) entfernt und durch das SalI/XbaI tag-54 Fragment ersetzt (Fig. 1). Die vollständige Expressionskassette im Pflanzenexpressionsvektor pSSH1 (Kay R. et al., Science 236 (1987), 1299-1302) enthielt den CaMV 35S Promoter mit dem doppelten Enhancer, die 5' nicht-translatierte Region der Chalconsynthase, die Signalpeptidsequenz der leichten Kette des monoklonalen Antikörpers 24 (Voss et al., Molecular Breeding 1 (1995), 39-50), den scFv24 (Zimmermann et. al, supra), den tag-54, der über einen Glycin-Glycin-Glycin-Glycin-Serin Linker im Leseraster mit dem scFv24 fusioniert war, und die 3' nicht-translatierte Region sowie Terminationssequenz von CaMV. Dieses Konstrukt, pscFv24-54tag, ermöglichte einen Transport und Akkummulation des rekombinanten Proteins in Apoplasten der Pflanzenzelle.

Ein zweiter Pflanzenexpressionsvektor, pscFv24-54tagK, der am C-Terminus der kodierenden Region zusätzlich die Aminosäuresequenz KDEL enthielt, ermöglichte die Anreicherung des rekombinanten Proteins im endoplasmatischen Retikulum der Pflanzenzelle.
Beide Konstrukte wurden transient in Nicotiana tabaccum cv. Petite Havanna SR1 Blättern transformiert und nach dreitägiger Inkubation wurden die löslichen Proteine durch Verreiben der Tabakblätter in Mörsern im Extraktionspuffer (200mM Tris-HCl pH 7.5, 5mM EDTA, Tween 20, 0.1mM DTT) isoliert.

Die Expression und Anreicherung der rekombinanten Proteine wurde mittels ELISA und Western-Blot über den Tag-54 mittels des Antikörpers mAk 54-1 nachgewiesen. Im ELISA konnten für das Konstrukt pscFv24-54tag 1375ng und für das Konstrukt pscFv24-54tagK 26480 ng funktionaler scFv24 pro Gramm Blattmaterial erfaßt werden.

Im ELISA wurden die folgenden Komponenten in der angegebenen Reihenfolge verwendet: 1) polyklonale Huhn-anti TMV Antikörper (41mg/ml 1:5,000 verdünnt), 2) 1µg/ml TMV vulgare, 3) aus transformierten Tabakblättern isoliertes lösliches Protein, 4) polyklonale Kaninchen Antikörper, die spezifisch an den monoklonalen Antikörper 24 binden (1:5,000 verdünnt), 5) mit alkalischer Phosphatase markierter Ziege-anti Kaninchen Antikörper (0.6mg/ml 1:5,000 verdünnt), 6) Phosphatasesubstrat p-Nitrophenyl Phosphat.

Im Western-Blot konnten beide rekombinanten Proteine, scFv24-54tag und scFv24-54tagK, spezifisch detektiert werden. Dazu wurden 20µl der aus den Tabakblättern isolierten löslichen Proteine in einem SDS-Polyacrylamidgel aufgetrennt, auf eine Nitrocellulosemembran übertragen (Maniatis, supra) und die rekombinanten Proteine scFv24-54tag und scFv24-54tagK wie folgt nachgewiesen: 1) mAk 54-1 (0.8mg/ml und 1:5,000 verdünnt), 2) ein mit alkalischer Phosphatase markierter Ziege-anti Maus Antikörper (0.6mg/ml 1:5,000 verdünnt), 3) Färbereagenz NBT/BCIP.

### b. Expression in Bakterien

Der Vektor pET22b (Novagen, Ca, USA) wurde derart modifiziert, so dass er eine "in-frame" Klonierung des GST-Proteins in Fusion zum tag-54 ermöglicht (Fig. 2). Zuerst wurde das tag54-Epitop in den bakteriellen Expressionsvektor pGEX5x-3 (Pharmacia, SE) kloniert und als GST (Glutathion-S-transferase) Fusionspeptid (GST-tag54) mittels transformierter E. coli BL21 Zellen exprimiert. Hierzu wurden die E. coli Zellen bei 30°C bis zu einer OD₆₀₀ₙₘ von 0,8 unter Schütteln angezogen und anschließend mit 1 mM IPTG induziert. Nach 4 h wurden die Zellen durch Zentrifugation (4000 x g, 4°C) gewonnen. Die Zellen wurden mittels Ultraschall lysiert worauf 30 µg Protein A gereinigter monoklonaler Antikörper mAK 54-1 zu 10 ml bakteriellem Lysat gegeben wurde

Nach Inkubation über 20 min wurde das gebildete GST-Tag54/mAK 54-1 Assoziationsprodukt mittels einer GST Affinitätsreinigung isoliert (Pharmacia, SE) und von der Matrix mittels reduziertem Glutathion eluiert. Der Komplex wurde mittels SDS-PAA Gelelektrophorese gelelektrophoretisch aufgetrennt, wobei auf dem Coomasie-Blau gefärbtem Gel sowohl die Antikörper leichte und schwere Kette als auch das GST-tag54 Protein nachgewiesen wurden.

## Patentansprüche

1. Polypeptid-Tag, das die folgende Aminosäuresequenz aufweist
**Lys-Asp-Trp-Glu-His-Leu**
bzw. eine von der vorstehend angegebenen Sequenz durch Ersatz einer Aminosäure abgeleiteten Sequenz, bei der die dreidimensionale bzw. Tertiärstruktur im wesentlichen erhalten bleibt.

2. Polypeptid-Tag nach Anspruch 1, das die folgenden Aminosäuresequenz aufweist
**Lys-His-Ile-Lys-Asp-Trp-Glu-His-Leu-Glu-Glu-Phe**
bzw. eine von der vorstehend angegebenen Sequenz durch Ersatz einer Aminosäure abgeleiteten Sequenz, bei der die dreidimensionale bzw. Tertiärstruktur im wesentlichen erhalten bleibt.

3. Expressionsvektor, kodierend ein Fusionspolypeptid, das ein gewünschtes Polypeptid und ein Polypeptid-Tag nach einem der Ansprüche 1 oder 2 umfaßt.

4. Expressionsvektor nach Anspruch 3, wobei das Polypeptid-Tag von dem gewünschten Polypeptid durch einen Abstandshalter getrennt ist.

5. Expressionsvektor nach Anspruch 3 oder 4, wobei das Polypeptid-Tag von der folgenden DNA-Sequenz kodiert ist:
**AAG GAC TGG GAG CAC**
bzw. einer davon abweichenden DNA-Sequenz, die ein Polypeptid-Tag nach Anspruch 1 kodiert.

6. Expressionsvektor nach Anspruch 3 oder 4, wobei das Polypeptid-Tag von der folgenden DNA-Sequenz kodiert ist:
**AAG CAC ATC AAG GAC TGG GAG CAC CTC GAA GAG TTC**
bzw. einer davon abweichenden DNA-Sequenz, die ein Polypeptid-Tag nach Anspruch 2 kodiert.

7. Expressionsvektor nach einem der Ansprüche 3 bis 6, wobei das Polypeptid-Tag am N-oder C-Terminus oder innerhalb des Polypeptids vorliegt.

8. Fusionspolypeptid, das von einem Expressionsvektor nach einem der Ansprüche 3 bis 7 kodiert ist.

9. Antikörper oder ein Fragment davon, der gegen ein Polypeptid-Tag nach einem der Ansprüche 1 oder 2 gerichtet ist.

10. Antikörper nach Anspruch 9, wobei der Antikörper monoklonal ist.

11. Verwendung eines Polypeptid-Tags nach Anspruch 1 oder 2 zur Isolierung und/oder zum Nachweis eines Polypeptids.

12. Verwendung eines Expressionsvektors nach einem der Ansprüche 3 bis 7 zur Herstellung und/oder Isolierung eines Polypeptids.

13. Verwendung eines Antikörpers nach Anspruch 9 oder 10 zur Reinigung und/oder Isolierung und/oder zum Nachweis eines Polypeptids.

14. Verwendung eines Antikörpers nach einem der Ansprüche 9 oder 10 zur Diagnostik von TMV-Infektionen.

15. Kit, umfassend, einen Expressionsvektor nach einem der Ansprüche 3 bis 7 und ggf. einen Antikörper nach einem der Ansprüche 9 oder 10.

## Claims

1. A polypeptide tag, having the following amino acid sequence
**Lys-Asp-Trp-Glu-His-Leu**
and, a sequence derived from the above specified sequence by replacement of an amino acid, respectively, in which the three dimensional and ternary structure, respectively, is essentially maintained.

2. A polypeptide tag according to claim 1, having the following amino acid sequence
**Lys-His-Ile-Lys-Asp-Trp-Glu-His-Leu-Glu-Glu-Phe**
and, a sequence derived from the above specified sequence by replacement of an amino acid, respectively, in which the three dimensional and ternary structure, respectively, is essentially maintained.

3. An expression vector coding a fusion polypeptide, comprising a desired polypeptide and a polypeptide tag according to any of the claims 1 or 2.

4. The expression vector according to claim 3, wherein said polypeptide tag is separated by a spacer from said desired polypeptide.

5. The expression vector according to claim 3 or 4, wherein said polypeptide tag is coded by the following DNA-sequence
**AAG GAC TGG GAG CAC**
and, by a DNA-sequence differing therefrom, respectively, coding a polypeptide tag according to claim 1.

6. The expression vector according to claim 3 or 4, wherein said polypeptide tag is coded by the following DNA-sequence:
**AAG CAC ATC AAG GAC TGG GAG CAC CTC GAA GAG TTC**
and, by a DNA-sequence differing therefrom, respectively, coding a polypeptide tag according to claim 2.

7. The expression vector according to any of the claims 3 to 6, wherein said polypeptide tag is at the N- or C-terminal end or within said polypeptide.

8. A fusion polypeptide, coded by an expression vector according to any of the claims 3 to 7.

9. An antibody or fragment thereof, directed against a polypeptide tag according to any of the claims 1 or 2.

10. The antibody according to claim 9, wherein said antibody is monoclonal.

11. Use of a polypeptide tag according to claim 1 or 2 for isolating and/or detecting of a polypeptide.

12. Use of an expression vector according to any of the claims 3 to 7 for producing and/or isolating of a polypeptide.

13. Use of an antibody according to claim 9 or 10 for purifying and/or isolating and/or detecting of a polypeptide.

14. Use of an antibody according to any of the claims 9 or 10 for diagnosis of TMV-infections.

15. A kit comprising an expression vector according to any of the claims 3 to 7 and, where required, an antibody according to any of the claims 9 or 10.

## Revendications

1. « Tag » polypeptidique qui présente la séquence d'acides aminés suivante
Lys - Asp - Trp - Glu - His - Leu
respectivement une séquence dérivée de la séquence indiquée précédemment par remplacement d'un acide aminé, dans laquelle la structure tridimensionnelle respectivement tertiaire demeure substantiellement conservée.

2. « Tag » polypeptidique selon la revendication 1 qui présente la séquence d'acides aminés suivante
Lys - His - Ile - Lys - Asp - Trp - Glu - His - Leu - Glu - Glu - Phe
respectivement une séquence dérivée de la séquence indiquée précédemment par remplacement d'un acide aminé, dans laquelle la structure tridimensionnelle, respectivement tertiaire demeure substantiellement conservée.

3. Vecteur d'expression codant un polypeptide de fusion qui recouvre un polypeptide désiré ou un « tag » polypeptidique selon l'une des revendications 1 ou 2.

4. Vecteur d'expression selon la revendication 3 dans lequel le « tag » polypeptidique est séparé du polypeptide désiré par un support de distanciation.

5. Vecteur d'expression selon la revendication 3 ou 4 dans lequel le « tag » polypeptidique est codé par la séquence ADN suivante :
AAG GAC TGG GAG CAC
respectivement une séquence ADN divergente qui code un « tag » polypeptidique selon la revendication 1.

6. Vecteur d'expression selon la revendication 3 ou 4 dans lequel le « tag » polypeptidique est codé par la séquence ADN suivante :
AAG CAC ATC AAG GAC TGG GAG CAC CTC GAA GAG TTC
respectivement une séquence ADN divergente qui code un « tag » polypeptidique selon la revendication 2.

7. Vecteur d'expression selon l'une des revendications 3 à 6 dans lequel le « tag » polypeptidique est présent à l'extrémité terminale N ou C ou à l'intérieur du polypeptide.

8. Polypeptide de fusion qui est codé par un vecteur d'expression selon l'une des revendications 3 à 7.

9. Anticorps ou fragment de celui-ci qui est dirigé contre un « tag » polypeptidique selon l'une des revendications 1 ou 2.

10. Anticorps selon la revendication 9 dans lequel l'anticorps est monoclonal.

11. Utilisation d'un « tag » polypeptidique selon la revendication 1 ou 2 pour l'isolation et/ou la détection d'un polypeptide.

12. Utilisation d'un vecteur d'expression selon l'une des revendications 3 à 7 pour la préparation et/ou l'isolation d'un polypeptide.

13. Utilisation d'un anticorps selon la revendication 9 ou 10 pour la purification et/ou la détection d'un polypeptide.

14. Utilisation d'un anticorps selon la revendication 9 ou 10 pour le diagnostic de l'infection « TMV ».

15. Trousse comprenant un vecteur d'expression selon l'une des revendications 3 à 7 et le cas échéant un anticorps selon l'une des revendications 9 ou 10.


### SEQUENZPROTOKOLL

<110> Fraunhofer Gesellschaft
<120> Polypeptid-Tags
<130> 80239
<140>
   <141>
<160> 9
<170> PatentIn Ver. 2.1
<210> 1
   <211> 259
   <212> PRT
   <213> Tobacco mosaic virus
<400> 1
   Met Ala Gln Val Thr Leu Lys Glu Ser Gly Pro Gly Ile Leu Lys Pro
     1               5                  10                  15
   Ser Gln Thr Leu Ser Leu Thr Cys Ser Phe Ser Gly Phe Ser Leu Ser
                20                  25                  30
   Thr Ser Gly Met Gly Val Gly Trp Ile Arg Gln Pro Ser Gly Lys Gly
            35                  40                  45
   Leu Glu Trp Leu Ala His Ile Trp Trp Asp Asp Asp Lys Tyr Tyr Asn
        50                  55                  60
   Pro Ser Leu Arg Ser Gln Leu Thr Ile Ser Lys Asp Thr Ser Arg Asn
    65                  70                  75                  80
   Gln Val Phe Leu Arg Ile Thr Asn Val Asp Thr Ala Asp Thr Ala Thr
                    85                  90                  95
   Tyr Tyr Cys Ala Arg Gly Tyr Tyr Gly Asn Asp Ser Pro Phe Ala Tyr
               100                 105                 110
   Trp Gly Gln Gly Thr Leu Leu Thr Val Ser Ser Gly Ala Gly Pro Thr
           115                 120                 125
   Ser Gly Ser Gly Lys Pro Gly Pro Gly Glu Gly Ser Thr Lys Gly Ala
       130                 135                 140
   Pro Asp Ile Val Leu Ser Gln Ser Pro Lys Phe Met Ser Thr Ser Val
   145 150 155 160
   Gly Asp Arg Val Ser Ile Thr Cys Lys Ala Ser Gln Ile Val Arg Thr
   165 170 175
   Ala Val Ala Trp Phe Gln Gln Lys Pro Gly Gln Ser Pro Lys Ala Leu
   180 185 190
   Ile Tyr Leu Ala Ser Asn Arg His Thr Gly Val Pro Asp Arg Phe Thr
   195 200 205
   Gly Ser Gly Ser Gly Thr Asp Phe Thr Leu Thr Ile Ser Asn Val Gln
   210 215 220
   Ser Glu Asp Leu Ala Asp Tyr Phe Cys Leu Gln His Trp Asn Tyr Pro
   225 230 235 240
   Phe Thr Phe Gly Ser Gly Thr Lys Leu Glu Ile Lys Arg Ala Val Asp
   245 250 255
   Ala Ala Ala
<210> 2
   <211> 6
   <212> PRT
   <213> Tobacco mosaic virus
<400> 2
   Lys Asp Trp Glu His Leu
   1 5
<210> 3
   <211> 12
   <212> PRT
   <213> Künstliche Sequenz
<220>
<223> Beschreibung der künstlichen Sequenz:Beschreibung der künstlichen Sequenz :synthetisch
<400> 3
   Lys His Ile Lys Asp Trp Glu His Leu Glu Glu Phe
   1 5 10
<210> 4
   <211> 15
   <212> DNA
   <213> Tobacco mosaic virus
<400> 4
   aaggactggg agcac 15
<210> 5
   <211> 36
   <212> DNA
   <213> Tobacco mosaic virus
<400> 5
   aagcacatca aggactggga gcacctcgaa gagttc 36
<210> 6
   <211> 59
   <212> DNA
   <213> Künstliche Sequenz
<220>
<223> Beschreibung der künstlichen Sequenz: synhetisch
<400> 6
   catgccatga ctcgcggccc agccggccat ggcccaggtt actctraaag wgtstggcc 59
<210> 7
   <211> 21
   <212> DNA
   <213> Künstliche Sequenz
<220>
   <223> Beschreibung der künstlichen Sequenz: synhetisch
<400> 7
   tgmrgagacg gtgaccagrg tc 21
<210> 8
   <211> 50
   <212> DNA
   <213> Künstliche Sequenz
<220>
   <223> Beschreibung der künstlichen Sequenz: synhetisch
<400> 8
   ctagtggtac tccacgcggc cgcgtcgaca gcmcgtttca gytccarytt 50
<210> 9
   <211> 48
   <212> DNA
   <213> Künstliche Sequenz
<220>
   <223> Beschreibung der künstlichen Sequenz: synhetisch
<400> 9
   catgccatga ctcgcggcgc gcctgacatt gtgmtgwchc agtctcca 48
<210> 4
   <211> 15
   <212> DNA
   <213> Tobacco mosaic virus
<400> 4
   aaggactggg agcac 15
<210> 5
   <211> 36
   <212> DNA
   <213> Tobacco mosaic virus
<400> 5
   aagcacatca aggactggga gcacctcgaa gagttc 36
<210> 6
   <211> 59
   <212> DNA
   <213> Künstliche Sequenz
<220>
   <223> Beschreibung der künstlichen Sequenz: synhetisch
<400> 6
   catgccatga ctcgcggccc agccggccat ggcccaggtt actctraaag wgtstggcc 59
<210> 7
   <211> 21
   <212> DNA
   <213> Künstliche Sequenz
<220>
   <223> Beschreibung der künstlichen Sequenz: synhetisch
<400> 7
   tgmrgagacg gtgaccagrg tc 21
<210> 8
   <211> 50
   <212> DNA
   <213> Künstliche Sequenz

### SEQUENZPROTOKOLL

<110> Fraunhofer Gesellschaft
<120> Polypeptid-Tags
<130> 80239
<140>
   <141>
<160> 9
<170> PatentIn Ver. 2.1
<210> 1
   <211> 259
   <212> PRT
   <213> Tobacco mosaic virus
<400> 1
   Met Ala Gln Val Thr Leu Lys Glu Ser Gly Pro Gly Ile Leu Lys Pro
    1               5                  10                  15
   Ser Gln Thr Leu Ser Leu Thr Cys Ser Phe Ser Gly Phe Ser Leu Ser
               20                  25                  30
   Thr Ser Gly Met Gly Val Gly Trp Ile Arg Gln Pro Ser Gly Lys Gly
           35                  40                  45
   Leu Glu Trp Leu Ala His Ile Trp Trp Asp Asp Asp Lys Tyr Tyr Asn
       50                  55                  60
   Pro Ser Leu Arg Ser Gln Leu Thr Ile Ser Lys Asp Thr Ser Arg Asn
   65                  70                  75                  80
   Gln Val Phe Leu Arg Ile Thr Asn Val Asp Thr Ala Asp Thr Ala Thr
                   85                  90                  95
   Tyr Tyr Cys Ala Arg Gly Tyr Tyr Gly Asn Asp Ser Pro Phe Ala Tyr
               100                 105                 110
   Trp Gly Gln Gly Thr Leu Leu Thr Val Ser Ser Gly Ala Gly Pro Thr
           115                 120                 125
   Ser Gly Ser Gly Lys Pro Gly Pro Gly Glu Gly Ser Thr Lys Gly Ala
       130                 135                 140
   Pro Asp Ile Val Leu Ser Gln Ser Pro Lys Phe Met Ser Thr Ser Val
   145 150 155 160
   Gly Asp Arg Val Ser Ile Thr Cys Lys Ala Ser Gln Ile Val Arg Thr
   165 170 175
   Ala Val Ala Trp Phe Gln Gln Lys Pro Gly Gln Ser Pro Lys Ala Leu
   180 185 190
   Ile Tyr Leu Ala Ser Asn Arg His Thr Gly Val Pro Asp Arg Phe Thr
   195 200 205
   Gly Ser Gly Ser Gly Thr Asp Phe Thr Leu Thr Ile Ser Asn Val Gln
   210 215 220
   Ser Glu Asp Leu Ala Asp Tyr Phe Cys Leu Gln His Trp Asn Tyr Pro
   225 230 235 240
   Phe Thr Phe Gly Ser Gly Thr Lys Leu Glu Ile Lys Arg Ala Val Asp
   245 250 255
   Ala Ala Ala
<210> 2
   <211> 6
   <212> PRT
   <213> Tobacco mosaic virus
<400> 2
   Lys Asp Trp Glu His Leu
   1 5
<210> 3
   <211> 12
   <212> PRT
   <213> Künstliche Sequenz
<220>
<223> Beschreibung der künstlichen Sequenz:Beschreibung der künstlichen Sequenz :synthetisch
<400> 3
   Lys His Ile Lys Asp Trp Glu His Leu Glu Glu Phe
   1 5 10
<210> 4
   <211> 15
   <212> DNA
   <213> Tobacco mosaic virus
<400> 4
   aaggactggg agcac 15
<210> 5
   <211> 36
   <212> DNA
   <213> Tobacco mosaic virus
<400> 5
   aagcacatca aggactggga gcacctcgaa gagttc 36
<210> 6
   <211> 59
   <212> DNA
   <213> Künstliche Sequenz
<220>
   <223> Beschreibung der künstlichen Sequenz: synhetisch
<400> 6
   catgccatga ctcgcggccc agccggccat ggcccaggtt actctraaag wgtstggcc 59
<210> 7
   <211> 21
   <212> DNA
   <213> Künstliche Sequenz
<220>
   <223> Beschreibung der künstlichen Sequenz: synhetisch
<400> 7
   tgmrgagacg gtgaccagrg t 21
<210> 8
   <211> 50
   <212> DNA
   <213> Künstliche Sequenz
<220>
   <223> Beschreibung der künstlichen Sequenz: synhetisch
<400> 8
   ctagtggtac tccacgcggc cgcgtcgaca gcmcgtttca gytccarytt 50
<210> 9
   <211> 48
   <212> DNA
   <213> Künstliche Sequenz
<220>
   <223> Beschreibung der künstlichen Sequenz: synhetisch
<400> 9
   catgccatga ctcgcggcgc gcctgacatt gtgmtgwchc agtctcca 48